# FASCICULE DE BREVET EUROPEEN

(11) **EP 4 276 923 B1**
(45) Date de publication et mention de la délivrance du brevet: **17.04.2024**
(21) Numéro de dépôt: 22315101.0
(22) Date de dépôt: 10.05.2022
(51) Int. Cl.: H10N 30/30, H10N 30/88, H10N 30/03, H02N 2/18, A61N 1/378

(54) **RÉCUPÉRATEUR D'ÉNERGIE PIÉZOÉLECTRIQUE AVEC LAME À DÉBATTEMENT CONTRÔLÉ, NOTAMMENT POUR L'ALIMENTATION D'UNE CAPSULE CARDIAQUE AUTONOME LEADLESS**
PIEZOELEKTRISCHE ENERGIEGEWINNUNGSVORRICHTUNG MIT KONTROLLIERTER BALKENAUSLENKUNG, INSBESONDERE ZUR STROMVERSORGUNG EINER DRAHTLOSEN AUTONOMEN KARDIOKAPSEL
PIEZOELECTRIC ENERGY HARVESTER WITH CONTROLLED BEAM DISPLACEMENT, IN PARTICULAR FOR POWER SUPPLY OF A LEADLESS AUTONOMOUS CARDIAC CAPSULE

(43) Date de publication de la demande: 15.11.2023
(73) Titulaire: CAIRDAC, 92160 Antony (FR)
(72) Inventeur: Regnier, Willy, 91160 Longjumeau (FR); Dohin, Julien, 92170 Vanves (FR); Nguyen-Dinh, An, 37520 La Riche (FR)
(74) Mandataire: Dupuis-Latour, Dominique

(56) Documents cités:
- WO-A1-2018/122244
- FR-A1- 3 082 434

## Description

### CONTEXTE DE L'INVENTION

### Domaine de l'invention

L'invention concerne les récupérateurs d'énergie, également dénommés "harvesters" ou "scavengers", qui recueillent l'énergie mécanique résultant de mouvements divers qu'ils subissent et convertissent cette énergie mécanique en énergie électrique.

Elle concerne plus spécifiquement les récupérateurs de type dit "PEH" (*Piezoelectric Energy Harvester*), qui utilisent comme transducteur mécano-électrique une lame piézoélectrique oscillante couplée à une masse mobile inertielle.

L'invention sera décrite plus particulièrement dans une application de ces récupérateurs d'énergie à des dispositifs médicaux autonomes, notamment les dispositifs de type capsule implantable autonome, en particulier ceux de ces dispositifs qui sont destinés à être implantés dans une cavité cardiaque.

Cette application, si elle est particulièrement avantageuse, ne doit toutefois pas être considérée comme limitative de l'invention, dont les enseignements peuvent être appliqués à de nombreux autres types de dispositifs autonomes incorporant un récupérateur d'énergie de type PEH, que ces dispositifs soient implantables ou non, qu'il s'agisse de dispositifs médicaux ou non.

### Description de la technique antérieure

Dans le domaine des implants médicaux, les récents progrès en matière de miniaturisation de dispositifs actifs et les progrès des sciences du vivant permettent dorénavant le développement d'une grande variété de systèmes miniaturisés implantables totalement autonomes, à des fins de surveillance, de diagnostic ou de traitement. Ces dispositifs mettent en oeuvre des procédures d'implantation moins invasives, plus de confort, des performances accrues et souvent ouvrent l'accès à des nouveaux types de diagnostics et traitements.

Lorsqu'elle est appliquée au domaine des implants médicaux, l'invention concerne plus précisément ceux de ces implants qui incorporent un système d'autoalimentation comprenant un récupérateur d'énergie mécanique associé à un organe de stockage d'énergie intégré tel qu'une batterie rechargeable ou une capacité à hautes performances.

En effet, l'un des aspects critiques de ces dispositifs miniaturisés est celui de l'autonomie électrique. La durée de vie d'un tel implant étant d'environ 8 à 10 ans, compte tenu des très faibles dimensions il n'est pas possible d'utiliser une batterie conventionnelle, même à forte densité.

Le récupérateur d'énergie pallie cet inconvénient en recueillant l'énergie mécanique résultant des mouvements divers subis par le corps du dispositif implanté. Ces mouvements peuvent avoir pour origine un certain nombre de phénomènes se produisant par exemple au rythme des battements cardiaques tels que les secousses périodiques de la paroi sur laquelle est ancré l'implant, les vibrations des tissus cardiaques liées entre autres aux fermetures et ouvertures des valves cardiaques, ou encore les variations de débit du flux sanguin dans le milieu environnant, qui sollicitent l'implant et le font osciller au rythme des variations de débit. L'énergie mécanique recueillie par le récupérateur est convertie en énergie électrique (tension ou courant) au moyen d'un transducteur mécanoélectrique approprié, pour assurer l'alimentation des divers circuits et capteurs du dispositif et la recharge de l'organe de stockage d'énergie. Ce système d'alimentation permet au dispositif de fonctionner en parfaite autonomie électrique pendant toute sa durée de vie.

Cette technique de récupération d'énergie est particulièrement bien adaptée à l'alimentation des capsules autonomes implantées dépourvues de toute liaison physique à un dispositif distant. Ces capsules sont dénommées pour cette raison "capsules *leadless*", pour les distinguer des électrodes ou capteurs disposés à l'extrémité distale d'une sonde (*lead*) parcourue sur toute sa longueur par un ou plusieurs conducteurs reliés à un générateur connecté à l'extrémité opposée, proximale.

L'invention n'est toutefois pas limitée à un type particulier de capsule, ni même d'implant leadless, et elle est applicable indifféremment à de nombreux autres types de dispositifs autonomes, quelle que soit leur destination fonctionnelle, cardiaque ou autre, médicale ou non.

Dans le cas d'une application cardiaque, la capsule leadless surveille en continu le rythme du patient et délivre si nécessaire au coeur des impulsions électriques de stimulation, de resynchronisation et/ou de défibrillation en cas de troubles du rythme détecté par la capsule. La capsule comprend par ailleurs divers circuits électroniques, capteurs, etc. ainsi que des moyens émetteurs/récepteurs de communication sans fil pour l'échange de données à distance, le tout étant intégré dans un corps de très petites dimensions qui puisse être implanté dans des sites difficilement accessibles ou laissant peu d'espace, tels que l'apex du ventricule, la paroi interne de l'oreillette, etc.

Le WO 2019/001829 A1 (Cairdac) décrit un exemple d'une telle capsule leadless intracardiaque.

L'invention concerne plus précisément les capsules ou dispositifs implantables analogues dont le récupérateur d'énergie est du type PEH, c'est-à-dire utilisant un transducteur piézoélectrique ou "PZT" (*PieZoelectric Transducer*) et un ensemble pendulaire inertiel soumis aux sollicitations externes décrites plus haut. L'ensemble pendulaire inertiel comprend une masse mobile logée dans le corps de la capsule, dite "masse sismique" où "masse inertielle", qui est entrainée au gré des mouvements de la capsule soumise en permanence aux diverses sollicitations externes décrites plus haut. Après chacune de ces sollicitations, la masse inertielle, qui est couplée à un élément élastiquement déformable, oscille à une fréquence propre d'oscillation libre.

L'énergie mécanique de l'oscillation est convertie en énergie électrique par un transducteur mécanoélectrique produisant un signal électrique. Ce transducteur mécanoélectrique peut en particulier être un PZT sollicité de façon cyclique en flexion de manière à engendrer au sein du matériau qui le constitue des charges électriques qui sont récupérées en surface du composant pour être utilisées par le système d'autoalimentation de la capsule leadless. Le PZT se présente le plus souvent sous la forme d'une lame encastrée à l'une de ses extrémités et couplée à la masse inertielle à son autre extrémité, qui est libre.

Le signal électrique en sortie du transducteur est délivré à un circuit de gestion de l'alimentation de la capsule, qui redresse et régule le signal électrique pour délivrer en sortie une tension ou un courant continus stabilisés permettant d'assurer l'alimentation des divers circuits électroniques et capteurs de la capsule, ainsi que la recharge de l'organe de stockage d'énergie.

La structure mécanique d'un tel récupérateur d'énergie de type PEH est notamment décrite en détail dans le WO 2018/122244 A1 (Sorin CRM/Regnier). Le US 2019/381325 A1 (Regnier et al., correspondant au FR 3 082 434 A1 (Cairdac)) décrit également une pièce multifonction assurant entre autres le positionnement et le maintien des différents éléments de l'ensemble pendulaire.

On notera que le terme de "lame" doit être entendu dans son sens le plus large, à savoir une bande mince et plate de forme allongée, étant entendu que la forme de cette bande n'est pas nécessairement rectangulaire ni son épaisseur constante (comme dans la description du mode de réalisation particulier qui sera donnée plus. bas). Le terme de "lame" au sens de la présente invention recouvre ainsi des éléments pouvant présenter une largeur et/ou une épaisseur qui ne sont pas constantes en direction longitudinale, ainsi qu'éventuellement une déformabilité pouvant aller au-delà d'un unique degré de liberté en flexion.

Le problème de l'invention réside dans la difficulté qu'il y a de bénéficier de l'amplitude maximale potentiellement disponible d'oscillation du PZT. Cette amplitude d'oscillation conditionne la quantité d'énergie effectivement récupérable par le PEH. En effet, les charges qui sont produites par la flexion de la lame et récupérées par le circuit de gestion et de régulation du PEH sont d'autant plus élevées que cette flexion est importante, et donc que le débattement de l'ensemble pendulaire est élevé.

Toutefois, pour garantir une longévité suffisante compte tenu notamment de la durée de vie d'un implant cardiaque (au moins dix ans sans défaillance), cette déformation, et donc l'amplitude d'oscillation de l'ensemble pendulaire, est restreinte à une valeur assurant un fonctionnement sans fatigue des matériaux du PZT même sur le très long terme.

Concrètement, dans l'exemple d'un PEH intégré à une capsule leadless dont la structure est comparable à celle décrite dans les WO 2018/122244 A1 et US 2019/381325 A1 précités, la course maximale de la masse inertielle est limitée à une valeur (au niveau de son centre de gravité) inférieure à 1,5 mm avec une précision d'environ 0,1 mm, pour ne pas pénaliser la fiabilité à très long terme de l'implant.

Le problème de l'invention vise plus spécifiquement la préservation de cette amplitude maximale d'oscillation en évitant qu'elle ne soit réduite par un positionnement sous-optimal de l'ensemble pendulaire dans le corps du module PEH au moment de l'assemblage de ce module, notamment du fait d'un positionnement imparfait de la masse inertielle.

En effet, les techniques connues d'assemblage de modules PEH telles que celles décrites dans les WO 2018/122244 A1 et US 2019/381325 A1 précités peuvent conduire à de légers décentrages ou désalignements de la masse inertielle dans son logement au moment du montage de l'ensemble pendulaire (lame PZT munie à une extrémité de sa pièce d'encastrement et à l'autre extrémité de la masse inertielle) dans le logement support du module PEH, avec une réduction corrélative de la course maximale de la masse inertielle et par voie de conséquence de l'amplitude de flexion du PZT et donc de la quantité de charges électriques récupérables produites par ce dernier.

Concrètement, on a pu constater qu'une perte de 0,1 mm, voire même de seulement 50 µm, sur une course maximale de 0,8 mm de la masse inertielle entraîne une perte notable de la puissance totale récupérée par le PEH pour l'alimentation des circuits de l'implant.

Le but de l'invention est de proposer une nouvelle structure de module PEH, et un nouveau procédé d'assemblage d'une telle structure, qui minimisent les pertes sur l'amplitude maximale d'oscillation du PZT, et permette donc d'obtenir la puissance maximale potentiellement disponible du PEH avec un centrage strict de la masse inertielle dans son logement support.

### RÉSUMÉ DE L'INVENTION

Pour résoudre ces problèmes et atteindre les buts exposés ci-dessus, l'invention propose un module PEH comprenant, de façon en elle-même connue notamment d'après le WO 2018/122244 A1 précité, un tube enveloppe allongé et, logé à l'intérieur du tube, un ensemble pendulaire comprenant : une lame de transducteur piézoélectrique, PZT, la lame s'étendant en direction axiale entre une extrémité proximale encastrée et une extrémité distale libre et étant élastiquement déformable en flexion ; une masse inertielle, couplée à l'extrémité distale libre de la lame et mobile en direction transversale à l'intérieur du tube ; et une monture de lame, apte à être solidarisée au tube et fixée à une pièce d'encastrement de lame à l'extrémité proximale de la lame. L'ensemble pendulaire est apte à convertir une énergie mécanique produite par des oscillations de l'ensemble pendulaire sous l'effet de sollicitations externes subies par le module en un signal électrique oscillant recueilli par des électrodes de surface de la lame.

De façon caractéristique de l'invention, ce module comprend en outre un insert de symétrisation, pour le calibrage et la symétrisation en direction transversale et latérale des oscillations de l'ensemble pendulaire, l'insert de symétrisation étant agencé à l'intérieur du tube dans une région de libre débattement de la lame à proximité de la masse inertielle. L'insert de symétrisation est distinct de la monture de lame, et il comprend : une partie périphérique apte à être solidarisée au tube indépendamment de la monture de lame, le tube présentant une forme intérieure conjuguée de la partie périphérique à l'endroit où doit être solidarisée cette dernière ; et une partie centrale avec une cavité axiale traversante à l'intérieur de laquelle s'étend la lame dans ladite région de libre débattement, la cavité axiale s'étendant entre des surfaces de limitation de course en vis-à-vis, les surfaces de limitation de course étant symétriques et étant aptes à venir en contact avec la lame dans une configuration de flexion de celle-ci.

Selon diverses formes de réalisation avantageuses :
- l'insert de symétrisation comprend un élément en matériau synthétique comprenant ladite partie centrale de l'insert, et un élément en matériau métallique apte à être soudé au tube au niveau de ladite partie périphérique de l'insert, les éléments en matériau synthétique et en matériau métallique étant mécaniquement solidarisés entre eux ;
- ladite partie périphérique de l'insert de symétrisation comprend un méplat (68) pour le positionnement en rotation axiale de l'insert de symétrisation par rapport au tube, et le tube comprend une surface conjuguée du méplat de l'insert ;
- l'insert de symétrisation comprend au moins une encoche de maintien d'un bord d'au moins une carte de circuit imprimé supportée par la monture de lame et par l'insert de symétrisation ; et/ou
- les surfaces de limitation de course de la partie centrale de l'insert de symétrisation présentent entre elles un écartement transversal variable, croissant dans un sens allant en éloignement de la monture de lame.

L'invention a également pour objet un procédé d'assemblage d'un tel module PEH, comprenant les étapes suivantes :
a) préparation d'un premier sous-ensemble comprenant une lame de transducteur piézoélectrique avec une masse inertielle à une extrémité distale de la lame et une pièce d'encastrement à une extrémité proximale ;
b) préparation d'un second sous-ensemble comprenant i) une monture de lame apte à recevoir la pièce d'encastrement, ii) un insert de symétrisation situé à distance axiale de la monture de lame, et iii) au moins une carte de circuit imprimé agencée entre la monture de lame et l'insert de symétrisation ;
c) préparation d'un troisième sous-ensemble par mise en place du premier sous-ensemble préparé à l'étape a) dans le second sous-ensemble préparé à l'étape b), avec fixation de la pièce d'encastrement à la monture de lame ;
d) mise en place du troisième sous-ensemble dans le tube ;
e) centrage transversal et latéral de la masse inertielle dans l'insert de symétrisation ;
f) assujettissement définitif au tube de l'insert de symétrisation ;
g) assujettissement définitif au tube de la monture de lame ; et
h) centrage du transducteur piézoélectrique par rapport au tube et assujettissement de la pièce d'encastrement (24) à la monture de lame (54).

Selon diverses formes de mise en oeuvre avantageuses de ce procédé :
- les assujettissements définitifs effectués aux étapes f), g) et/ou h) sont des soudages ;
- le procédé comprend une étape, préalable à l'étape b), d'obtention de l'insert de symétrisation par solidarisation mécanique d'un élément en matériau synthétique avec un élément en matériau métallique apte à être ultérieurement soudé au tube à l'étape f) ; et/ou
- dans ce dernier cas, la solidarisation mécanique est un bouterollage d'un pion de l'élément en matériau synthétique dans un alésage de l'élément en matériau métallique.

L'invention porte également sur un dispositif autonome incorporant dans un corps de dispositif un module PEH tel que ci-dessus et comprenant :
- un ensemble électronique ;
- un module PEH tel que ci-dessus, produisant en sortie un signal électrique ;
- un circuit de gestion d'alimentation, apte à redresser et réguler le signal électrique produit par le module PEH, pour délivrer en sortie une tension ou un courant continus d'alimentation stabilisés ; et
- un organe de stockage d'énergie pour l'alimentation de l'ensemble électronique,

Ladite tension ou ledit courant continus stabilisés délivrés par le circuit de gestion d'alimentation servent à l'alimentation de l'ensemble électronique et/ou à la recharge de l'organe de stockage d'énergie du dispositif autonome.

Ce dispositif autonome peut notamment être un dispositif médical actif de type capsule leadless, comprenant un corps de capsule avec un élément d'ancrage à une paroi d'un organe d'un patient, et dans laquelle les sollicitations externes auxquelles est soumis l'ensemble pendulaire du module PEH sont des sollicitations appliquées au corps de capsule sous l'effet de mouvements de ladite paroi et/ou de variations de débit d'un flux dans le milieu environnant.

### DESCRIPTION SOMMAIRE DES DESSINS

On va maintenant décrire un exemple de réalisation de la présente invention en référence aux dessins annexés, où les mêmes références désignent d'une figure à l'autre des éléments identiques ou fonctionnellement semblables.
La Figure 1 illustre des dispositifs médicaux de type capsule leadless dans leur environnement, avec divers exemples de sites d'implantation dans, sur ou à proximité du coeur d'un patient.
La Figure 2 illustre une capsule leadless implantée au fond du ventricule droit d'un patient.
La Figure 3 montre isolément un ensemble pendulaire de type connu, avec un PZT en forme de lame allongée encastrée à une extrémité et supportant une masse inertielle à son extrémité opposée.
La Figure 4 présente 'sous forme schématique les principaux blocs fonctionnels constitutifs d'une capsule leadless.
La Figure 5 est une vue en coupe transversale, par un plan axial, du module PEH selon l'invention.
La Figure 6 est une vue éclatée en perspective montrant les différents éléments constitutifs du module PEH de la Figure 5.
La Figure 7 est une vue perspective, de trois-quarts avant, montrant isolément l'insert de symétrisation caractéristique du module PEH selon l'invention.
Les Figures 8 et 9 sont des vues perspectives, respectivement de trois-quarts avant et de trois-quarts arrière, de la pièce en matériau synthétique constituant l'un des éléments de l'insert de symétrisation illustré Figure 7.
Les Figures 10 et 11 sont des vues de face du module PEH selon l'invention illustré Figure 5, respectivement en configuration de position neutre de la lame et en configuration de flexion maximale de la lame.
Les Figures 12 et 13 illustrent deux étapes préalables du processus d'assemblage du module PEH selon l'invention.
La Figure 14 illustre, en coupe, le module obtenu à l'issue des étapes illustrées Figures 12 et 13, avant l'exécution des soudages finals du processus d'assemblage.
La Figure 15 illustre le dispositif obtenu en fin de processus, après adjonction des pièces complémentaires permettant d'obtenir une capsule leadless implantable.
La Figure 16 est un organigramme explicitant les différentes étapes du processus d'assemblage illustré aux Figures 12 à 15.

### DESCRIPTION DÉTAILLÉE DE MODES DE RÉALISATION PRÉFÉRENTIELS DE L'INVENTION

On va maintenant décrire un exemple de réalisation du dispositif de l'invention, dans une application à une capsule implantable autonome destinée à être implantée dans une cavité cardiaque.

Comme on l'a indiqué plus haut, cette application particulière n'est donnée qu'à titre d'exemple de réalisation et n'est pas limitative de l'invention, dont les enseignements peuvent être appliqués à de nombreux autre types de dispositifs autonomes incorporant un récupérateur d'énergie de type PEH, que ces dispositifs soient implantables ou non, qu'il s'agisse de dispositifs médicaux ou non.

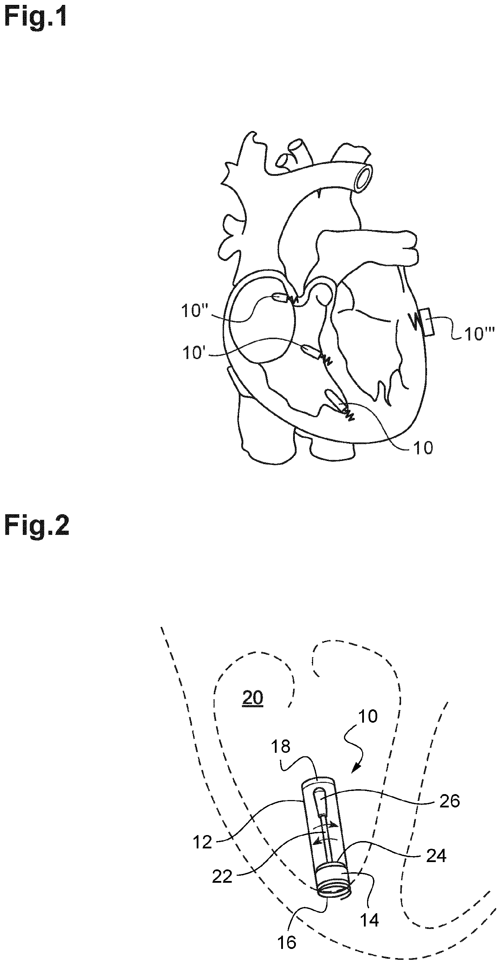

Sur la Figure 1, on a représenté diverses possibilités de sites d'implantation d'un dispositif de type leadless, dans une application, à la stimulation cardiaque. Ainsi, la capsule 10 est implantée à l'intérieur d'une cavité du myocarde (implant endocavitaire), par exemple à l'apex du ventricule droit. En variante, la capsule peut être également implantée sur le septum interventriculaire droit, comme en 10', ou encore sur une paroi auriculaire, comme illustré en 10". Le dispositif peut également être une capsule épicardique placée sur une région externe du myocarde, comme illustré en 10‴.

Dans chaque cas, la capsule leadless est fixée à la paroi cardiaque au moyen d'un système d'ancrage saillant pénétrant dans le tissu cardiaque pour le maintien sur le site d'implantation. D'autres systèmes d'ancrage sont utilisables, et ne modifient en aucune façon la mise en oeuvre de la présente invention.

La capsule 10 se présente sous forme extérieure d'un implant avec un corps tubulaire allongé 12 enfermant les divers circuits électroniques et d'alimentation de la capsule ainsi qu'un récupérateur d'énergie à ensemble pendulaire. Les dimensions typiques des capsules connues sont un diamètre de l'ordre de 6 mm pour une longueur d'environ 25 à 40 mm.

Le corps tubulaire 12 comporte à son extrémité avant (distale) 14 un élément d'ancrage saillant, par exemple une vis hélicoïdale 16 pour maintenir la capsule sur le site d'implantation. D'autres systèmes d'ancrage sont utilisables, et ne modifient en aucune façon la mise en oeuvre de la présente invention. L'extrémité opposée (proximale) 18 de la capsule 10 est une extrémité libre, qui est seulement pourvue de moyens (non représentés) de liaison temporaire à un cathéter-guide ou autre accessoire d'implantation utilisé pour la mise en place ou l'explantation de la capsule, qui est ensuite désolidarisé de cette dernière.

Dans l'exemple illustré Figure 2, la capsule leadless 10 est un implant endocavitaire implanté à l'intérieur d'une cavité 20 du myocarde, par exemple à l'apex du ventricule droit. En variante, toujours dans une application à la stimulation cardiaque, la capsule peut être également implantée sur le septum interventriculaire ou sur une paroi auriculaire, ou encore être une capsule épicardique placée sur une région externe du myocarde, ces différents modes d'implantation ne modifiant en aucune façon la mise en oeuvre de la présente invention. Pour assurer les fonctions de détection/stimulation, une électrode (non représentée) en contact avec le tissu cardiaque au site d'implantation assure le recueil des potentiels de dépolarisation cardiaque et/ou l'application d'impulsions de stimulation. Dans certaines formes de réalisation, la fonction de cette électrode peut être assurée par la vis d'ancrage 16, qui est alors une vis active, électriquement conductrice et reliée au circuit de détection/stimulation de la capsule.

La capsule leadless 10 est par ailleurs dotée d'un module récupérateur d'énergie dit "PEH", comprenant un ensemble pendulaire inertiel qui oscille à l'intérieur de la capsule au gré des diverses sollicitations externes auxquelles la capsule est soumise. Ces sollicitations peuvent notamment résulter : des mouvements de la paroi à laquelle est ancrée la capsule, qui sont transmis au corps tubulaire 12 par la vis d'ancrage 16 ; et/ou des variations du débit du flux sanguin dans le milieu environnant la capsule, qui produisent des oscillations du corps tubulaire 12 au rythme des battements cardiaques ; et/ou des vibrations diverses transmises par les tissus cardiaques.

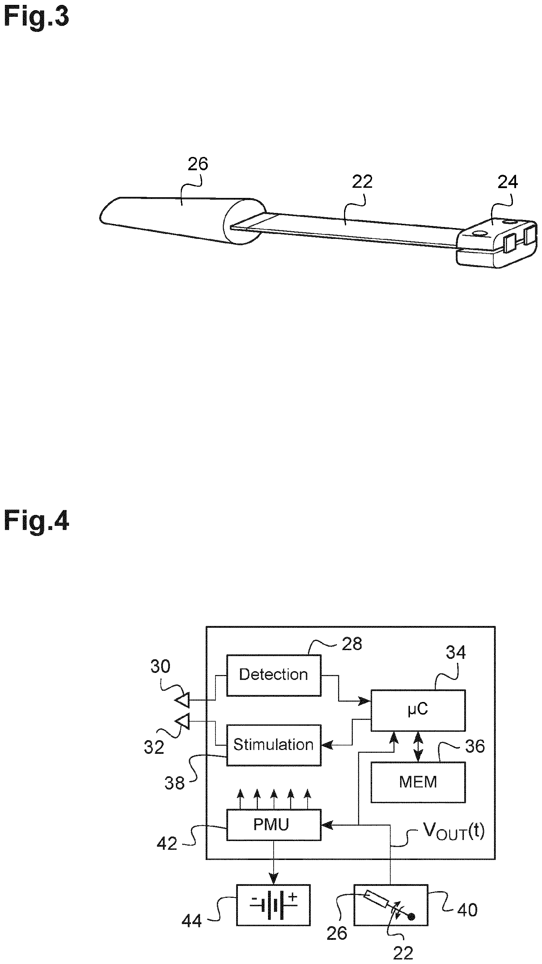

L'ensemble pendulaire, illustré isolément Figure 3, est constitué d'une lame piézoélectrique 22 assujettie à une pièce d'encastrement 24 à l'une de ses extrémités (ci-après "extrémité proximale" de la lame) et dont l'extrémité opposée, libre (ci-après "extrémité distale" de la lame) est couplée à une masse inertielle mobile 26. La lame piézoélectrique 22 est une lame flexible élastiquement déformable qui constitue avec la masse inertielle 26 un système pendulaire de type masse-ressort. Du fait de son inertie, la masse 26 soumet la lame 22 à une déformation de type vibratoire de part et d'autre d'une position neutre ou non déformée correspondant à une position stable de repos en l'absence de toute sollicitation. Les dimensions typiques minimales des lames PZT des dispositifs connus de ce type sont de l'ordre de 25 mm de long pour une largeur de 5 mm environ.

De fait, pour ce qui est de son comportement mécanique, cet ensemble peut être assimilé à une structure de type "poutre encastrée-libre", présentant une fréquence d'oscillation propre qui est ici la fréquence sur laquelle oscille le système masse-ressort. On notera que cette fréquence d'oscillation propre, typiquement de l'ordre de quelques dizaines de hertz, est notablement supérieure à la fréquence des sollicitations cycliques externes qui correspondent à la fréquence des battements cardiaques (quelques hertz tout au plus). Ainsi, à chaque contraction cardiaque la masse inertielle (ou autre organe mécanique fonctionnellement analogue) sera sollicitée avec une amplitude plus ou moins importante, puis le système pendulaire oscillera plusieurs fois avec des amplitudes décroissantes (rebonds caractéristiques d'une oscillation périodique amortie), et enfin se stabilisera jusqu'au battement cardiaque suivant, où le cycle sollicitation/oscillations se reproduira de façon comparable.

La lame 22 assure en outre, par effet piézoélectrique, une fonction de transducteur mécanoélectrique permettant de convertir en charges électriques la contrainte mécanique de flexion qui lui est appliquée. Ces charges sont collectées par des électrodes à la surface de la lame de manière à produire un signal électrique qui, après redressement, stabilisation et filtrage, alimentera les divers circuits électroniques de la capsule.

La Figure 4 est un synoptique des divers circuits électriques et électroniques intégrés à la capsule leadless, présenté sous forme de blocs fonctionnels.

Le bloc 28 désigne un circuit de détection de l'onde de dépolarisation cardiaque, qui est relié à une électrode de cathode 30 en contact avec le tissu cardiaque et à une électrode d'anode 32 associée, par exemple une électrode annulaire formée sur le corps tubulaire de la capsule. Le bloc de détection 28 comprend des filtres et des moyens de traitement analogique et/ou numérique du signal recueilli. Le signal ainsi traité est appliqué à l'entrée d'un microcalculateur 34 associé à une mémoire 36. L'ensemble électronique comporte également un circuit de stimulation 38 opérant sous le contrôle du microcalculateur 34 pour délivrer au système d'électrodes 30, 32 des impulsions de stimulation du myocarde.

Il est par ailleurs prévu un circuit récupérateur d'énergie ou PEH 40, constitué par l'ensemble pendulaire formé par la lame piézoélectrique 22 et la masse inertielle 26 décrits plus haut en référence aux Figures 2 et 3. Comme la lame piézoélectrique 22 assure aussi une fonction de transducteur mécano-électrique, elle convertit en charges électriques les sollicitations mécaniques subies et produit un signal électrique variable V_{OUT}(t), qui est un signal alternatif oscillant à la fréquence d'oscillation libre de l'ensemble pendulaire lame 22/masse 26, et au rythme des battements successifs du myocarde auquel la capsule est couplée.

Le signal électrique variable Vouï(t) est délivré à un circuit de gestion de l'énergie ou PMU 42. Le PMU 42 redresse et régule le signal V_{OUT}(t) de manière à produire en sortie une tension ou un courant continus stabilisés servant à l'alimentation des divers circuits électroniques et à la recharge de la batterie intégrée 44.

D'autre part, la lame est avantageusement une lame de type bimorphe, c'est-à-dire capable de générer de l'énergie sur ses deux faces lorsqu'elle est soumise à une déformation. Ces propriétés de transduction sont typiques d'un matériau piézoélectrique tel que les céramiques PZT ou les monocristaux de type PMN-PT, titanate de baryum ou niobate de lithium.

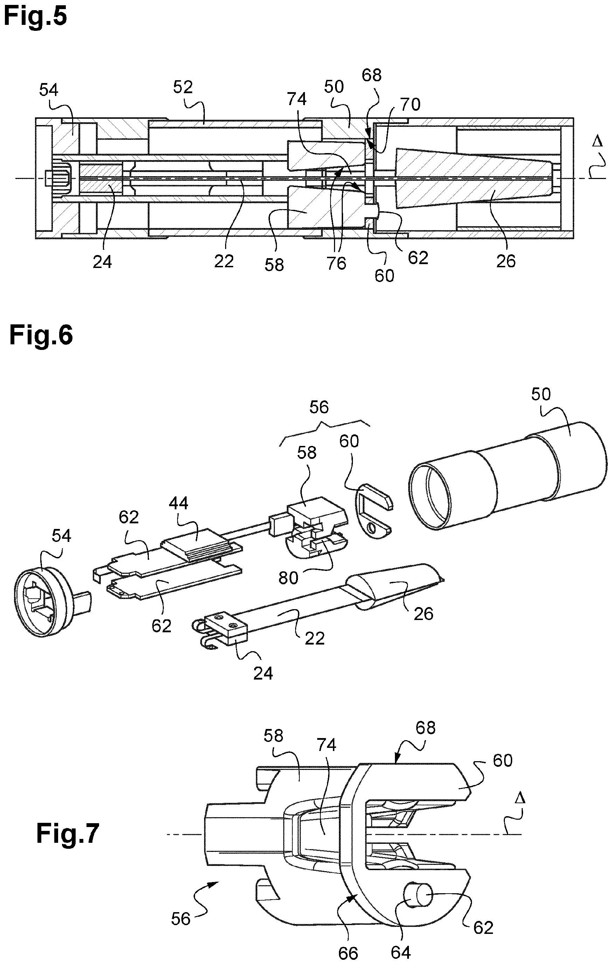

Sur les Figures 5 et 6 on a représenté les principaux éléments constitutifs d'un module PEH selon l'invention.

Ces différents éléments sont logés à l'intérieur d'un tube enveloppe 50, qui est généralement un tube métallique (pour permettre des opérations de soudage qui seront décrites plus bas), de préférence en titane en raison de l'excellente biocompatibilité de ce métal.

Un tube enveloppe particulièrement adapté à la réalisation d'une capsule leadless est décrit notamment dans le EP 3 730 185 A1 (Cairdac), correspondant au US 2020/338241 A1 (Regnieret al.), qui illustre notamment un tube composite métal/céramique comprenant une partie centrale (52 sur la Figure 5) en matériau céramique transparent aux radiofréquences, de manière à permettre une communication sans fil entre des circuits électroniques situés à l'intérieur du tube et l'environnement extérieur, le reste du tube étant en matériau métallique tel que le titane et le tout formant un ensemble monobloc tubulaire.

Le tube enveloppe 50 loge l'ensemble pendulaire constitué de la lame 22 maintenue côté proximal par la pièce d'encastrement 24 et portant côté distal la masse inertielle 26. L'ensemble pendulaire est agencé au centre du tube enveloppe 50 et aligné sur l'axe Δ du tube.

Dans la suite, on entendra par "direction axiale" la direction de plus grande longueur de la lame et par "direction transversale" la direction de déformation de la lame, direction qui est située dans un plan radial et qui est perpendiculaire à la direction axiale Δ ; la direction perpendiculaire aux directions axiale et transversale sera dénommée "direction latérale".

La pièce d'encastrement 24 est maintenue dans le tube par une monture 54 assujettie au tube, notamment une monture en un matériau métallique tel que le titane, susceptible d'être soudé au tube en périphérie de manière à assujettir au tube 50 la monture 54, et donc l'encastrement 24 et la lame 22.

Le EP 3 892 325 A1 (Cairdac), correspondant au US 2021/316148 A1 (Regnier et al.), décrit en détail un exemple de pièce d'encastrement et de monture, et on pourra se référer à ce document pour de plus amples détails.

Du côté distal de la lame, avant la masse inertielle 26 et à proximité de celle-ci est agencée une pièce caractéristique de la présente invention, ci-après désignée "insert de symétrisation".

L'insert de symétrisation 56 est une pièce distincte de la pièce d'encastrement 24 et de la monture 54, à la différence notamment de la pièce multifonction décrite dans le US 2019/381325 A1 précité, où ces deux éléments appartiennent à une même pièce monobloc en matériau synthétique.

L'insert de symétrisation 56 est constitué de deux éléments 58, 60, à savoir une pièce 58 en matériau synthétique et une pièce 60 en matériau métallique qui seront décrits plus en détail ci-après en référence aux Figures 7 à 9.

Le tube 50 loge également une ou plusieurs cartes de circuit imprimé (PCB) 62, dans l'exemple illustré deux PCB 62 dont l'un porte la batterie 44. Ces deux PCB sont reliés l'un à l'autre par une nappe de conducteurs flexibles et supportés à chacune de leurs extrémités respectivement côté distal par l'insert 56 et côté proximal par la monture 54.

La configuration de ces PCB de part et d'autre de la lame 22, et la manière dont ils sont réunis par une nappe flexible et supportés entre un élément proximal et un élément distal sont décrits notamment dans le US 2019/381325 A1 précité, auquel on pourra se référer pour de plus amples détails.

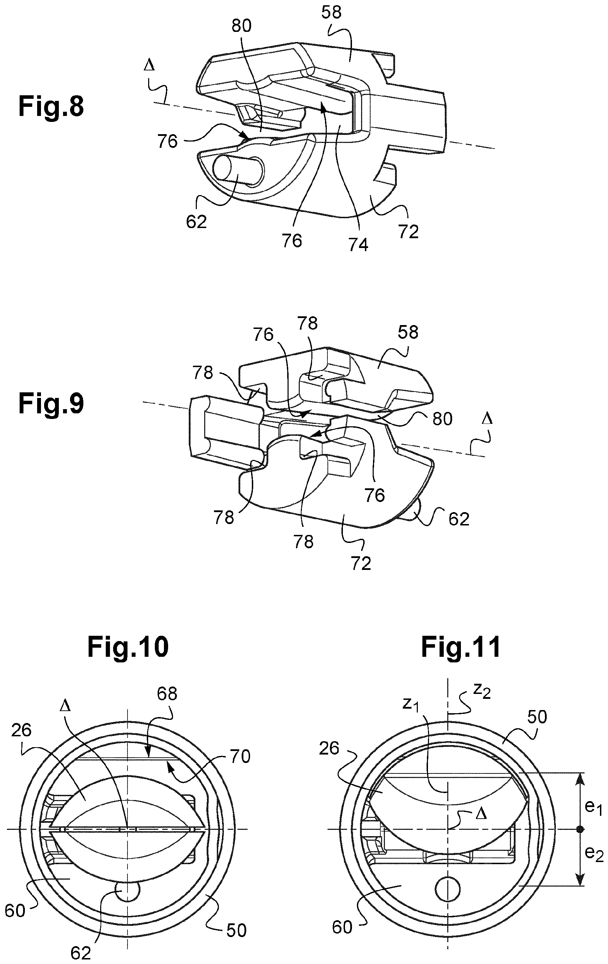

Les Figures 7 à 9 illustrent de façon plus détaillée l'insert de symétrisation 56 caractéristique de l'invention. La Figure 7 montre isolément en perspective cet insert avec les deux éléments 58 et 60 respectivement en matériau synthétique et en matériau métallique réunis ensemble, tandis que les Figures 8 et 9 montrent isolément la pièce 58 en matériau synthétique, en perspective de trois-quarts avant et de trois-quarts arrière.

Le matériau synthétique de la pièce 58 est un matériau diélectrique tel qu'un polymère thermoplastique de polyuréthane de type *Tecothane*^{®} ou un polymère de type PET (poly(téréphtalate d'éthylène)) ou PEEK (polyétheréthercétone), ou autre matière plastique injectable, ou réalisée par d'autres techniques, notamment par impression 3D à partir d'une résine polymère diélectrique.

Le matériau métallique de la pièce 60 est avantageusement le titane, mais ce peut être également un acier inoxydable, par exemple un acier 316L, ou encore un métal tel que le tantale ou un alliage nickel-titane de type nitinol.

Les deux pièces 58 et 60, qui sont en matériaux de natures différentes, sont montées ensemble et assemblées par un procédé mécanique, par exemple par bouterollage d'un pion 62 de la pièce 58 en matériau synthétique, inséré dans un alésage 64 de la pièce 60 en matériau métallique.

La pièce 60 en matériau métallique présente une surface périphérique externe 66 dimensionnée de manière à pouvoir être assemblée sans jeu à l'intérieur d'une région homologue du tube métallique 50 dont la surface intérieure est conjuguée de la forme de la surface périphérique 66 de la pièce 60. La pièce 60 comporte également un méplat 68 pour le positionnement en rotation axiale de la pièce 60 à l'intérieur du tube, ce méplat 68 coopérant avec une surface interne de forme conjuguée 70 du tube 50.

Une fois les pièces 58 et 60 mécaniquement assemblées entre elles, la pièce 60 en matériau métallique, et par voie de conséquence la pièce 58 en matériau synthétique, pourra être précisément ajustée à l'intérieur du tube, à la fois en rotation axiale (grâce au méplat 68) et dans les directions transversale et latérale (par la coopération sans jeu entre la surface extérieure 66 de la pièce 60 et la surface intérieure conjuguée cylindrique du tube 50).

La pièce 58 en matériau synthétique; illustrée isolément et plus en détail sur les Figures 8 et 9, comprend un corps 72 définissant une cavité centrale axiale traversante 74 au travers de laquelle passera la lame 22 en configuration finale assemblée (cf. Figure 5).

La cavité 74 est délimitée, de part et d'autre de l'axe Δ, par deux surfaces de limitation de course 76 s'étendant en vis-à-vis, symétriquement par rapport à un plan axial perpendiculaire à la direction transversale (la direction verticale sur la coupe de la Figure 5). Les deux surfaces de limitation de course symétriques présentent avantageusement un écartement variable en direction transversale, allant croissant dans une direction côté proximal vers côté radial de la lame.

Avantageusement, ces surfaces à écartement variable sont chacune des surfaces sans discontinuité en direction axiale et définissent dans un plan axial une courbure continûment variable homologue d'une courbure que présente la lame au fur et à mesure de sa flexion. Cette configuration de surfaces de limitation de course permet de déplacer progressivement, dans un sens en éloignement de la pièce d'encastrement, le point de contact de la lame 22 contre la surface de limitation de course 76, et de réduire ainsi la longueur libre de la lame au fur et à mesure de la flexion de cette dernière.

Les avantages d'un tel agencement sont décrits en détail et quantifiés notamment dans le US 2019/381325 A1 précité, où cette géométrie est décrite sous la désignation de "bec de canard".

La pièce 58 en matériau synthétique comporte également une encoche 78 destinée à supporter les PCB 62 de chaque côté de l'axe principal Δ. La pièce 58 comporte également une fente 80 donnant accès à la cavité axiale 74 par le côté, de façon à permettre l'introduction dans cette cavité de la lame 22 déjà munie de son encastrement 24 et de sa masse inertielle 26 (c'est-à-dire de la lame dans la configuration de la Figure 6).

Les Figures 10 et 11 sont des vues de face du module PEH selon l'invention dans son état final assemblé, correspondant à la coupe de la Figure 5, respectivement en configuration de position neutre de la lame (avec la lame 22 et la masse inertielle 26 s'étendant le long de l'axe Δ) et en configuration de.flexion maximale de la lame 22, dont la partie libre située juste avant la masse inertielle 26 vient en contact avec l'une des surfaces de limitation de course 76.

Lorsque la lame se déforme sous l'effet des sollicitations appliquées de l'extérieur au module PEH, les axes respectifs transversaux *z₁* et *z₂* de la masse inertielle 26 et du tube 50 restent alignés et confondus.

Par ailleurs, l'insert de symétrisation 56 permet, du fait du placement très précis de la pièce 60 en matériau métallique par rapport au tube 50, de positionner très précisément les deux surfaces de limitation de course 76 à des distances respectives égales *e₁* et *e₂* de l'axe Δ, ce qui garantit de pouvoir bénéficier de l'excursion maximale de la masse inertielle 26, et donc de la flexion maximale de la lame 22 lors des oscillations de l'ensemble pendulaire.

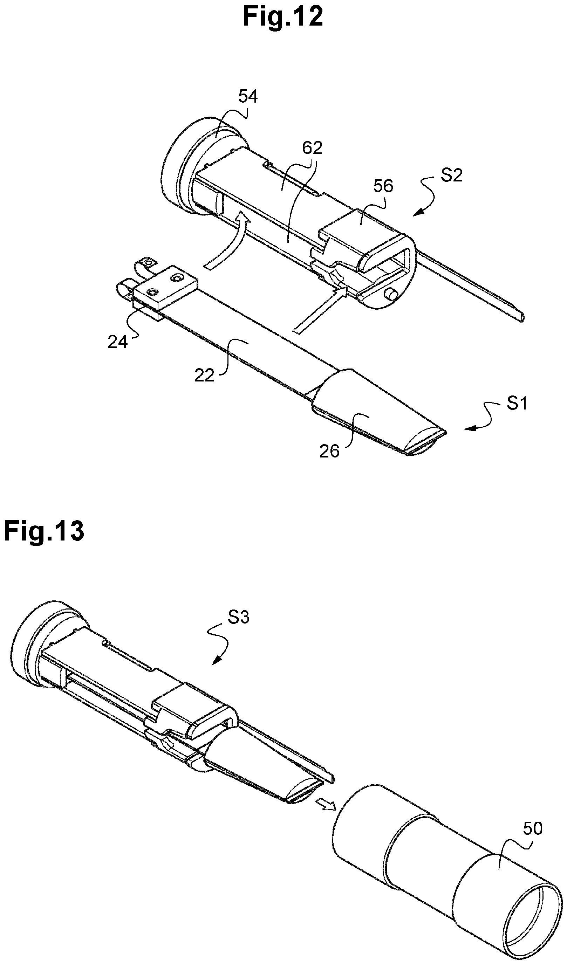

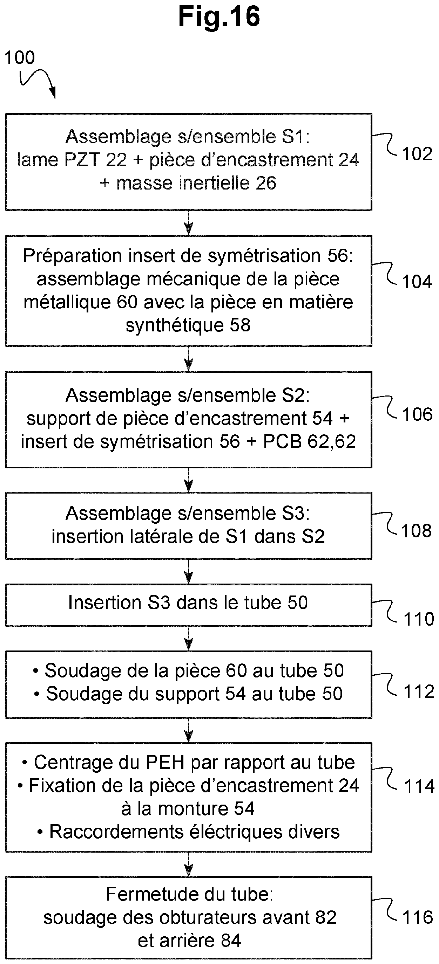

En référence aux Figures 12 à 15, on va maintenant exposer un procédé d'assemblage du module PEH que l'on vient de décrire, les différentes étapes de ce processus de fabrication étant explicitées sur l'organigramme de la Figure 16.

La première étape (bloc 102 de l'organigramme 100 de la Figure 16) consiste à assembler un premier sous-ensemble S1 (Figure 12) constitué de la lame PZT 22 avec la pièce d'encastrement 24 à son extrémité proximale et la masse inertielle 26 à son extrémité distale. Cette étape est réalisée par des techniques en elles-mêmes connues, par exemple celle décrite dans le EP 3 892 325 A1 précité pour l'encastrement 24, et pour la masse inertielle 26 par exemple par collage de deux pièces symétriques formant ensemble un tronc de cône, de part et d'autre de la lame 22 dans la partie libre de celle-ci.

L'étape suivante (bloc 104) consiste à préparer l'insert de symétrisation 56 par assemblage mécanique de la pièce 60 en matériau métallique sur la pièce 58 en matériau synthétique (Figure 7), par exemple par bouterollage du pion 62 de la pièce 60 dans l'alésage 64 de la pièce 58.

Une fois constitué l'insert de symétrisation 56, l'étape suivante (bloc 106) consiste à assembler un sous-ensemble S2 (Figure 12) regroupant la monture 54 et l'insert de symétrisation 56 avec les deux PCB 62 montés entre ces deux éléments 54 et 56. On notera que les PCB 62 sont simplement emboîtés à leurs extrémités sur les pièces 54 et 56 mais que, du fait du jeu d'assemblage résiduel et de la flexibilité propre des PCB, le sous-ensemble S2 n'est pas totalement rigide et laisse notamment subsister entre la monture 54 et l'insert de symétrisation 56 une légère marge de déformation relative en rotation et en translation transversale et latérale.

L'étape suivante (bloc 108) consiste à réunir les sous-ensembles S1 et S2 en un sous-ensemble S3, par introduction de la lame 22 dans la fente latérale 80 de l'insert de symétrisation 56 et de la pièce d'encastrement 24 dans la monture 54.

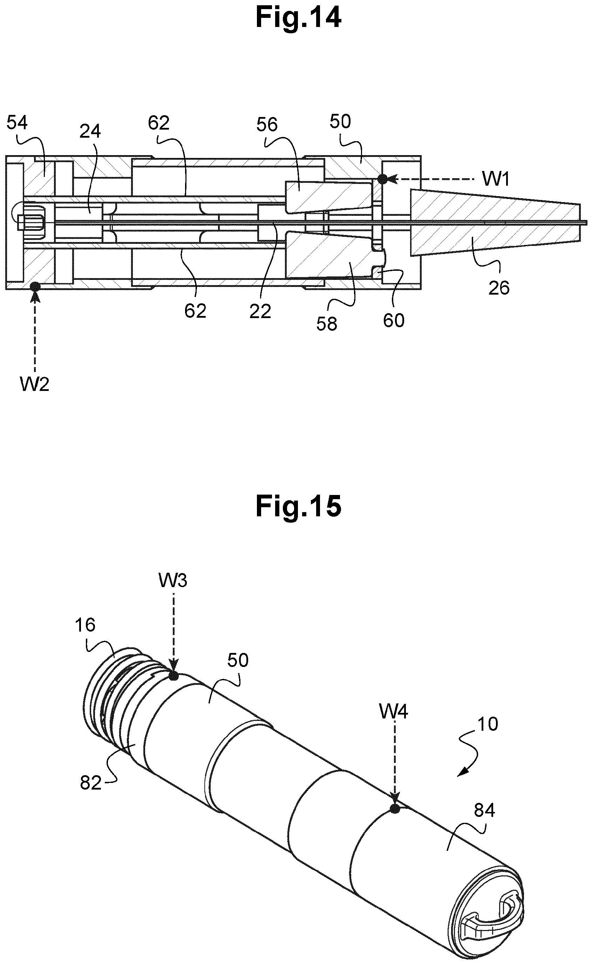

L'étape suivante (bloc 110) consiste à introduire ce sous-ensemble S3 dans le tube enveloppe 50 par translation axiale (Figure 13). Le résultat de cette étape est illustré, en coupe, sur la Figure 14.

L'étape suivante (bloc 112) consiste à centrer de façon précise l'insert de symétrisation 56 par rapport au tube 50, transversalement, latéralement, et en rotation, par un ajustement précis de la pièce 60 en matériau métallique de l'insert de symétrisation 56 par rapport au logement de forme correspondante du tube enveloppe 50. Une fois ce centrage effectué, l'insert de symétrisation 56 est assujetti définitivement au tube 50, avantageusement par soudage (comme en W1) de la pièce métallique 60 sur le corps métallique du tube 50. Le soudage est réalisé par exemple en plusieurs points par des tirs laser dirigés vers l'interface entre ces deux pièces. A l'issue de cette opération, l'alignement précis de l'insert de symétrisation garantit le positionnement parfaitement symétrique des surfaces de limitation de course 76 de part et d'autre de l'axe Δ, et donc le débattement maximal de la lame lorsque celle-ci oscillera.

Une fois l'insert de symétrisation assujetti au tube, la monture 54 supportant la pièce d'encastrement 24 est assujettie au tube 50, ici encore de façon avantageuse par soudage (comme en W2) en plusieurs points au moyen de tirs laser périphériques.

Le fait que l'insert de symétrisation 56 et la monture 54 soient des pièces distinctes, et que l'insert 58 soit assujetti au tube avant la monture 54, fait en sorte qu'un éventuel désalignement entre ces deux pièces sera sans conséquence sur le centrage de la lame 22 et de la masse inertielle 26 par rapport au tube. Cette caractéristique de l'invention diffère des systèmes antérieurs tels que celui décrit par exemple dans le US 2019/381325 A1 précité, qui utilise une pièce multifonction unique en matériau synthétique, introduite à force dans le tube enveloppe métallique, assurant le positionnement et le maintien des différents éléments de l'ensemble pendulaire.

À l'étape suivante (bloc 114), le PEH est centré par rapport au tube 50, puis la pièce d'encastrement 24, et donc le sous-ensemble S1, est assujettie à la monture 54, par exemple par soudage. Cette étape comprend également diverses opérations annexes telles que notamment la réalisation de connexions électriques entre les différents éléments de l'implant (PCB 52, électrodes du PZT, etc.).

L'étape finale (bloc 116) consiste à fermer à ses deux extrémités le tube 50 contenant l'ensemble pendulaire, par ajout d'un obturateur avant 82 portant la vis d'ancrage 16 de la capsule leadless et d'un obturateur arrière 84. Ces obturateurs 82 et 84 sont assujettis au tube 50 par des soudages périphériques W3 et W4. La capsule leadless se présente alors dans son état assemblé final.

## Revendications

1. Un module de récupération d'énergie, PEH, comprenant :
- un tube enveloppe (50) allongé ;
- logé à l'intérieur du tube (50), un ensemble pendulaire comprenant :
. une lame (22) de transducteur piézoélectrique, PZT, la lame (22) s'étendant en direction axiale entre une extrémité proximale encastrée et une extrémité distale libre et étant élastiquement déformable en flexion ;
. une masse inertielle' (26), couplée à l'extrémité distale libre de la lame (22) et mobile en direction transversale à l'intérieur du tube (50) ; et
. une monture de lame (54), apte à être solidarisée au tube (50) et fixée à une pièce d'encastrement (24) de lame (22) à l'extrémité proximale de la lame (22),
l'ensemble pendulaire étant apte à convertir une énergie mécanique produite par des oscillations de l'ensemble pendulaire sous l'effet de sollicitations externes subies par le module en un signal électrique oscillant recueilli par des électrodes de surface de la lame,
**caractérisé en ce qu'**il comporte en outre :
- un insert de symétrisation (56), pour le calibrage et la symétrisation en direction transversale et latérale des oscillations de l'ensemble pendulaire, l'insert de symétrisation (56) étant agencé à l'intérieur du tube (50) dans une région de libre débattement de la lame (22) à proximité de la masse inertielle (26),
l'insert de symétrisation (56) étant distinct de la monture de lame (54) et comprenant :
. une partie périphérique (66) apte à être solidarisée au tube (50) indépendamment de la monture de lame (54), le tube (50) présentant une forme intérieure conjuguée de la partie périphérique (66) à l'endroit où doit être solidarisée cette dernière ; et
. une partie centrale avec une cavité axiale (74) traversante à l'intérieur de laquelle s'étend la lame (22) dans ladite région de libre débattement, la cavité axiale (74) s'étendant entre des surfaces de limitation de course (76, 76) en vis-à-vis, les surfaces de limitation de course (76, 76) étant symétriques et étant aptes à venir en contact avec la lame (22) dans une configuration de flexion de celle-ci.

2. Le module de la revendication 1, dans lequel l'insert de symétrisation (56) comprend :
- un élément en matériau synthétique (58) comprenant ladite partie centrale de l'insert (56) ; et
- un élément en matériau métallique (60) apte à être soudé au tube (50) au niveau de ladite partie périphérique (66) de l'insert (56),
les éléments en matériau synthétique (58) et en matériau métallique (60) étant mécaniquement solidarisés entre eux.

3. Le module de la revendication 1, dans lequel ladite partie périphérique (66) de l'insert de symétrisation (56) comprend un méplat (68) pour le positionnement en rotation axiale de l'insert de symétrisation (56) par rapport au tube (50), et le tube (50) comprend une surface (70) conjuguée du méplat (68) de l'insert (56).

4. Le module de la revendication 1, dans lequel l'insert de symétrisation (56) comprend au moins une encoche de maintien d'un bord d'au moins une carte de circuit imprimé (62, 62) supportée par la monture de lame (54) et par l'insert de symétrisation (56).

5. Le module de la revendication 1, dans lequel les surfaces de limitation de course (76, 76) de la partie centrale de l'insert de symétrisation (56) présentent entre elles un écartement transversal variable, croissant dans un sens allant en éloignement de la monture de lame (54).

6. Un procédé d'assemblage d'un module PEH selon l'une des revendications 1 à 5, **caractérisé en ce qu'**il comprend des étapes de :
a) préparation (102) d'un premier sous-ensemble (S1) comprenant une lame (22) de transducteur piézoélectrique avec une masse inertielle (26) à une extrémité distale de la lame (22) et une pièce d'encastrement (24) à une extrémité proximale ;
b) préparation (106) d'un second sous-ensemble (S2) comprenant i) une monture de lame (54) apte à recevoir la pièce d'encastrement (24), ii) un insert de symétrisation (56) situé à distance axiale de la monture de lame (54), et iii) au moins une carte de circuit imprimé (62, 62) agencée entre la monture de lame (54) et l'insert de symétrisation (56) ;
c) préparation (108) d'un troisième sous-ensemble (S3) par mise en place du premier sous-ensemble (S1) préparé à l'étape a) dans le second sous-ensemble (S2) préparé à l'étape b) ;
d) mise en place (110) du troisième sous-ensemble (S3) dans le tube (50) ;
e) centrage transversal et latéral de la masse inertielle (26) dans l'insert de symétrisation (56) ;
f) assujettissement définitif (112) au tube (50) de l'insert de symétrisation (56) ;
g) assujettissement définitif (112) au tube (50) de la monture de lame (54) ; et
h) centrage du transducteur piézoélectrique par rapport au tube (50) et assujettissement de la pièce d'encastrement (24) à la monture de lame (54).

7. Le procédé de la revendication 6, dans lequel les assujettissements définitifs effectués aux étapes f), g) et/ou h) sont des soudages (W1, W2).

8. Le procédé de la revendication 7, comprenant une étape (104), préalable à l'étape b), d'obtention de l'insert de symétrisation (56) par solidarisation mécanique d'un élément en matériau synthétique (58) avec un élément en matériau métallique (60) apte à être ultérieurement soudé au tube (50) à l'étape f).

9. Le procédé de la revendication 8, dans lequel la solidarisation mécanique est un bouterollage d'un pion (62) de l'élément en matériau synthétique (58) dans un alésage (64) de l'élément en matériau métallique (60).

10. Un dispositif autonome logeant, dans un corps de dispositif :
- un ensemble électronique (28-38) ;
- un module PEH (40) selon l'une des revendications 1 à 5, produisant en sortie un signal électrique ;
- un circuit de gestion d'alimentation (42), apte à redresser et réguler le signal électrique produit par le module PEH, pour délivrer en sortie une tension ou un courant continus d'alimentation stabilisés ; et
- un organe de stockage d'énergie (44) pour l'alimentation de l'ensemble électronique,
dans lequel ladite tension ou ledit courant continus stabilisés délivrés par le circuit de gestion d'alimentation servent à l'alimentation de l'ensemble électronique et/ou à la recharge de l'organe de stockage d'énergie du dispositif autonome.

11. Le dispositif autonome de la revendication 10,
dans lequel le dispositif autonome est un dispositif médical actif de type capsule autonome implantable (10) comprenant un corps de capsule (12) avec un élément d'ancrage (16) à une paroi d'un organe d'un patient,
et dans lequel les sollicitations externes auxquelles est soumis l'ensemble pendulaire (22, 26) du module PEH sont des sollicitations appliquées au corps de capsule (12) sous l'effet de mouvements de ladite paroi et/ou de variations de débit d'un flux dans le milieu environnant

## Patentansprüche

1. Ein Modul zur Energierückgewinnung, PEH, umfassend:
- ein langgezogenes Mantelrohr (50);
- im Inneren des Rohrs (50) aufgenommen eine Pendelanordnung, umfassend:
. eine Zunge (22) eines piezoelektrischen Wandlers, PZT, wobei sich die Zunge (22) in axialer Richtung zwischen einem verankerten proximalen Ende und einem freien distalen Ende erstreckt und durch Biegung elastisch verformbar ist;
. eine inertiale Masse (26), die mit dem freien distalen Ende der Zunge (22) gekoppelt und in Querrichtung im Inneren des Rohrs (50) beweglich ist; und
. eine Zungenhalterung (54), die imstande ist, mit dem Rohr (50) fest verbunden zu werden, und an einem Teil zur Verankerung (24) der Zunge (22) an dem proximalen Ende der Zunge (22) befestigt ist,
wobei die Pendelanordnung imstande ist, eine von Schwingungen der Pendelanordnung unter der Einwirkung von äußeren Belastungen, die das Modul erfährt, erzeugte mechanische Energie in eine elektrisches Schwingungssignal zu wandeln, das von Flächenelektroden der Zunge aufgenommen wird,
**dadurch gekennzeichnet, dass** es ferner aufweist:
- einen Symmetrierungseinsatz (56) für die Kalibrierung und die Symmetrierung in Querrichtung und in seitlicher Richtung der Schwingungen der Pendelanordnung, wobei der Symmetrierungseinsatz (56) im Inneren des Rohrs (50) in einer das freie Ausschlagen der Zunge (22) gestattenden Region in der Nähe der inertialen Masse (26) vorgesehen ist,
wobei der Symmetrierungseinsatz (56) von der Zungenhalterung (54) verschieden ist und Folgendes umfasst:
. einen umlaufenden Teil (66), der imstande ist, unabhängig von der Zungenhalterung (54) mit dem Rohr (50) fest verbunden zu werden, wobei das Rohr (50) eine innere Form aufweist, die an der Stelle, wo der umlaufende Teil (66) fest verbunden werden soll, an diesen letzten angepasst ist; und
. einen mittigen Teil mit einem durchgehenden axialen Hohlraum (74), in dessen Innerem sich die Zunge (22) in der das freie Ausschlagen gestattenden Region erstreckt, wobei sich der axiale Hohlraum (74) zwischen gegenüberliegenden Hubbegrenzungsflächen (76, 76) erstreckt, wobei die Hubbegrenzungsflächen (76, 76) symmetrisch sind und imstande sind, mit der Zunge (22) in einer Biegekonfiguration von dieser in Kontakt zu kommen.

2. Das Modul nach Anspruch 1, wobei der Symmetrierungseinsatz (56) Folgendes umfasst:
- ein Element (58) aus synthetischem Material, das den mittigen Teil des Einsatzes (56) umfasst; und
- ein Element (60) aus metallischem Material, das imstande ist, an das Rohr (50) im Bereich des umlaufenden Teils (66) des Einsatzes (56) geschweißt zu werden,
wobei das Element (58) aus synthetischem Material und das Element (60) aus metallischem Material mechanisch fest miteinander verbunden sind.

3. Das Modul nach Anspruch 1, wobei der umlaufende Teil (66) des Symmetrierungseinsatzes (56) eine Abflachung (68) für die Positionierung in axialer Rotation des Symmetrierungseinsatzes (56) relativ zu dem Rohr (50) umfasst und das Rohr (50) eine Fläche (70) aufweist, die an die Abflachung (68) des Einsatzes (56) angepasst ist.

4. Das Modul nach Anspruch 1, wobei der Symmetrierungseinsatz (56) wenigstens eine Einkerbung zum Festhalten eines Randes wenigstens einer gedruckten Leiterplatte (62, 62) umfasst, die von der Zungenhalterung (54) und von dem Symmetrierungseinsatz (56) getragen wird.

5. Das Modul nach Anspruch 1, wobei die Hubbegrenzungsflächen (76, 76) des mittigen Teil des Symmetrierungseinsatzes (56) zwischen einander einen variablen Querabstand aufweisen, der in einer Richtung, die von der Zungenhalterung (54) weg zeigt, zunimmt.

6. Ein Verfahren zum Montieren eines PEH-Moduls nach einem der Ansprüche 1 bis 5, **dadurch gekennzeichnet, dass** es die folgenden Schritte umfasst:
a) Vorbereiten (102) einer ersten Baugruppe (S1), umfassend eine Zunge (22) eines piezoelektrischen Wandlers mit einer inertialen Masse (26) an einem distalen Ende der Zunge (22) und ein Verankerungsteil (24) an einem proximalen Ende;
b) Vorbereiten (106) einer zweiten Baugruppe (S2), umfassend i) eine Zungenhalterung (54), die imstande ist, das Verankerungsteil (24) aufzunehmen, ii) einen Symmetrierungseinsatz (56), der sich in axialem Abstand von der Zungenhalterung (54) befindet, und iii) wenigstens eine gedruckte Leiterplatte (62, 62), die zwischen der Zungenhalterung (54) und dem Symmetrierungseinsatz (56) vorgesehen ist;
c) Vorbereiten (108) einer dritten Baugruppe (S3) durch Einbringen der in Schritt a) vorbereiteten ersten Baugruppe (S1) in die in Schritt b) vorbereitete zweite Baugruppe (S2);
d) Einbringen (110) der dritten Baugruppe (S3) in das Rohr (50);
c) Querzentrieren und seitliches Zentrieren der inertialen Masse (26) in dem Symmetrierungseinsatz (56);
f) endgültiges Festlegen (112) des Symmetrierungseinsatzes (56) an dem Rohr (50);
g) endgültiges Festlegen (112) der Zungenhalterung (54) an dem Rohr (50); und
h) Zentrieren des piezoelektrischen Wandlers relativ zu dem Rohr (50) und Festlegen des Verankerungsteils (24) an der Zungenhalterung (54) .

7. Das Verfahren nach Anspruch 6, bei dem das endgültigen Festlegen, das in den Schritten f), g) und/oder h) vorgenommen wird, Schweißungen (W1, W2) sind.

8. Das Verfahren nach Anspruch 7, umfassend einen Schritt (104), vor Schritt b), zum Herstellen des Symmetrierungseinsatzes (56) durch festes mechanisches Verbinden eines Elements (58) aus synthetischem Material mit einem Element (60) aus metallischem Material, der imstande ist, in Schritt f) später an das Rohr (50) geschweißt zu werden.

9. Das Verfahren nach Anspruch 8, bei dem das feste mechanische Verbinden ein Nietstempeln eines Stiftes (62) des Elements (58) aus synthetischem Material in einer Bohrung (64) des Elements (60) aus metallischem Material ist.

10. Eine autonome Vorrichtung, die in einem Vorrichtungskörper Folgendes aufnimmt:
- eine elektronische Anordnung (28-38);
- ein PEH-Modul (40) nach einem der Ansprüche 1 bis 5, das ausgangsseitig ein elektrisches Signal erzeugt;
- eine Versorgungsverwaltungsschaltung (42), die imstande ist, das von dem PEH-Modul erzeugte Signal gleichzurichten und zu regeln, um ausgangsseitig eine stabilisierte Vorsorgungsgleichspannung oder einen stabilisierten Versorgungsgleichstrom bereitzustellen; und
- ein Energiespeicherorgan (44) für die Versorgung der elektronischen Anordnung,
wobei die stabilisierte Gleichspannung oder der stabilisierte Gleichstrom, die von der Versorgungsverwaltungsschaltung bereitgestellt werden, der Versorgung der elektronischen Anordnung und/oder der Aufladung des Energiespeicherorgans der autonomen Vorrichtung dienen.

11. Die autonome Vorrichtung nach Anspruch 10, wobei die autonome Vorrichtung eine aktive medizinische Vorrichtung (10) vom Typ implantierbare autonome Kapsel ist, die einen Kapselkörper (12) mit einem Element (16) zur Verankerung an einer Wand eines Organs eines Patienten umfasst, und wobei die äußeren Belastungen, die die Pendelanordnung (22, 26) des PEH-Moduls erfährt, Belastungen sind, die auf den Kapselkörper (12) unter der Einwirkung von Bewegungen der Wand und/oder von Veränderungen einer Blutflussrate im umgebenden Medium aufgebracht werden.

## Claims

1. An energy harvesting module, PEH, comprising:
- an elongated envelope tube (50);
- contained inside the tube (50), a pendular unit comprising:
. a piezoelectric transducer, PZT, beam (22), the beam (22) extending in axial direction between a clamped proximal end and a free distal end and being elastically deformable in bending;
. an inertial mass (26), coupled to the free distal end of the beam (22) and mobile in transverse direction inside the tube (50); and
. a beam mount (54), adapted to be secured to the tube (50) and fastened to a clamping part (24) for the beam (22) at the proximal end of the beam (22),
wherein the pendular unit is adapted to convert a mechanical energy produced by oscillations of the pendular unit under the effect of external stresses undergone by the module into an oscillating electrical signal collected by surface electrodes of the beam,
**characterized by** further comprising:
- a symmetrization insert (56), for calibrating and symmetrizing in transverse and lateral directions the pendular unit oscillations, the symmetrization insert (56) being arranged inside the tube (50) in a region of free travel of the beam (22) near the inertial mass (26),
wherein the symmetrization insert (56) is distinct from the beam mount (54) and comprises:
. a peripheral portion (66) adapted to be secured to the tube (50) independently of the beam mount (54),
the tube (50) having an inner shape mating with the outer shape of the peripheral portion (66) at the place where the peripheral portion (66) has to be secured; and
. a central portion with an axial through-cavity (74) inside which the beam (22) extends in said region of free travel, the axial cavity (74) extending between opposite travel limitation surfaces (76, 76), the travel limitation surfaces (76, 76) being symmetrical and being capable of coming into contact with the beam (22) in a bending configuration of the latter.

2. The module of claim 1, wherein the symmetrization insert (56) comprises:
- a synthetic-material element (58) comprising said central portion of the insert (56); and
- a metal-material element (60) adapted to be welded to the tube (50) at said peripheral portion (66) of the insert (56),
wherein the synthetic-material (58) and metal-material (60) elements are mechanically secured to each other.

3. The module of claim 1, wherein said peripheral portion (66) of the symmetrization insert (56) comprises a flat surface (68) for the positioning in axial rotation of the symmetrization insert (56) with respect to the tube (50), and the tube (50) comprises a surface (70) mating with the flat surface (68) of the insert (56).

4. The module of claim 1, wherein the symmetrization insert (56) comprises at least one notch for holding an edge of at least one printed circuit board (62, 62) supported by the beam mount (54) and by the symmetrization insert (56).

5. The module of claim 1, wherein the travel limitation surfaces (76, 76) of the central portion of the symmetrization insert (56) have a mutual transverse spacing which increases in a direction away from the beam mount (54).

6. A method for assembling a PEH module according to any one of preceding claims 1-5, **characterized by** comprising steps of:
a) preparation (102) of a first sub-set (S1) comprising a piezoelectric transducer beam (22), with an inertial mass (26) at a distal end of the beam (22) and a clamping part (24) at a proximal end;
b) preparation (106) of a second sub-set (S2) comprising i) a beam mount (54) adapted to receive the clamping part (24), ii) a symmetrization insert (56) located at an axial distance from the beam mount (54), and iii) at least one printed circuit board (62, 62) arranged between the beam mount (54) and the symmetrization insert (56);
c) preparation (108) of a third sub-set (S3) by positioning the first sub-set prepared at step a) into the second sub-set prepared at step b);
d) positioning (110) of the third sub-set (S3) in the tube (50);
e) transverse and lateral centering of the inertial mass (26) in the symmetrization insert (56);
f) definitive attachment (112) of the symmetrization insert (56) to the tube (50);
g) definitive attachment (112) of the beam mount (54) to the tube (50); and
h) centering of the piezoelectric transducer with respect to the tube (50), and attachment of the clamping part (24) to the beam mount (54).

7. The method of claim 6, wherein the definitive attachments at steps f), g) and/or h) are weldings (W1, W2).

8. The method of claim 7, comprising a step (104), prior to step b), of obtaining the symmetrization insert (56) by mechanical securing of a synthetic-material element (58) to a metal-material element (60) adapted to be subsequently welded to the tube (50) at step f).

9. The method of claim 8, wherein the mechanical securing is a snap-riveting of a pin (62) of the synthetic-material element (58) into a bore (64) of the metal-material element (60).

10. An autonomous device further containing, in a device body:
- an electronic unit (28-38);
- a PEH module (40) according to one of claims 1-5, outputting an electrical signal;
- a power management circuit (42) adapted to rectify and regulate the electric signal produced by the PEH module, for outputting a stabilized direct power voltage or current; and
- an energy storage component (44) for powering the electronic unit,
wherein said stabilized direct voltage or current provided by the power management circuit are used to power the electronic unit and/or to charge the energy storage component of the autonomous device.

11. The autonomous device of claim 10,
wherein the autonomous device is an active medical device of the implantable autonomous capsule type (10), comprising a capsule body (12) with an element (16) for anchoring to a wall of a patient's organ,
and wherein the external stresses to which is subjected the pendular unit (22, 26) of the PEH module are stresses applied to the capsule body (12) resulting from movements of said wall and/or from flow rate variations of a flow in the surrounding environment.
